# EUROPEAN PATENT SPECIFICATION

(11) **EP 3 254 646 B1**
(45) Date of publication and mention of the grant of the patent: **15.08.2018**
(21) Application number: 16173142.7
(22) Date of filing: 06.06.2016
(51) Int. Cl.: A61F 2/40, A61F 2/30

(54) **ELLIPSOID SHAPED HUMERUS HEAD PROSTHESIS**
ELLIPSOIDFÖRMIGE HUMERUSKOPFPROTHESE
PROTHÈSE DE TÊTE HUMÉRALE EN FORME D'ELLIPSOÏDE

(43) Date of publication of application: 13.12.2017
(73) Proprietor: Habermeyer, Peter, 81925 München (DE)
(72) Inventor: Habermeyer, Peter, 81925 München (DE)
(74) Representative: Lohr, Georg

(56) References cited:
- DE-A1-102004 042 502
- US-A- 4 550 450
- US-A1- 2006 009 852
- US-A1- 2007 225 818
- US-A1- 2012 232 668
- US-A1- 2015 250 601

## Description

### Field of the invention

The invention relates to a shoulder joint prosthesis and in particular to a humerus implant.

### Description of the related art

The shoulder joint is a ball-and-socket-joint. It has an exceptional range of motion. A shoulder joint may be replaced or repaired if it suffers from instability or other maladies, such as arthrosis or fracture.

A humerus prosthesis is disclosed in US 2011/0320004 A1. The prosthesis has a shaft which is anchored into the humerus. The shaft holds a head which has a spherical cap.

WO 2005/070345 A1 discloses a shoulder joint prosthesis with a spherical cap attached to an anchoring section which is directly anchored into the bone of a humerus head. It does not require an elongated shaft anchored in the humerus. This design allows a simple, cement-free anchoring with excellent mechanical properties. The disadvantage is a higher wear of the articular cartilage of the glenoid. Literature reports a glenoid wear in 25% of patient after 4 years receiving Hemiarthroplasty.

US 2007/0225818 discloses a prior art humerus head implant of half-ellipsoid shape.

### Summary of the invention

The problem to be solved by the invention is to provide a humeral head prosthesis which can easily be implanted into the bone of the humerus and which provides a reduced wear of the articular cartilage of the glenoid. Furthermore, the implant should have high mechanical stability, a long lifetime, and should be manufactured at comparatively low costs.

Solutions of the problem are described in the independent claims. The dependent claims relate to further improvements of the invention.

In a first embodiment, the prosthesis comprises a head cap and a fixing component for fixing the head cap to the humerus head. The articulating surface of the head cap has the shape of a half ellipsoid and preferably of a half three-axial ellipsoid. The head cap preferably has a conical recess which matches to a conical section of the fixing component. This conical section of the fixing component is attached to a holding device, a bone screw which allows anchoring of the fixing component in the bone material of a humerus head without requiring a shank anchored in the humerus shaft.

The half-ellipsoid-shaped prosthesis head extends into the directions of an x-axis, a y-axis, and a z-axis. The z-axis is oriented towards the glenoid of a shoulder, while the x-axis is oriented in an anterior-posterior (transversal plane) direction. The y-axis is oriented craniocaudally (frontal plane) direction. The extension of the head cap is largest along the y-axis. In the direction of the x-axis, there is a smaller extension which is in a range of 0.8 to 0.95 of the extension in the y-axis. Most preferably, this ratio is between 0.85 and 0.95. A further preferred range is between 0.90 and 0.91. The extension in the direction of the z-axis is the smallest and is preferably below 50 percent of the extension in y-direction. The head cap has the shape of a half ellipsoid, as there is only an extension into positive z-direction, but no extension in a negative z-direction.

Furthermore, a center axis defined by the conical recess in the head cap has an offset to the z-axis of the half-ellipsoid. Most preferably, the center of the conical recess is below or in a negative y-direction related to the z-axis of the half-ellipsoid head cap. This displacement preferably is in a range of between 3 and 10 mm, and most preferably is in a range between 4 and 6 mm.

This results in a plurality of advantages over the prior art. Due to the ellipsoid shape, the contact area to a glenoid is significantly enlarged. This knowledge is the result of a large number of measurements with statistical evaluation which have been conducted at human shoulder joints. Due to the enlarged contact surface, the surface pressure is lowered, therefore decreasing wear of the cartilage and of the prosthesis head cap. This further increases lifetime of the prosthesis and increases shoulder revision intervals. The offset between the center of the conical recess and the z-axis of the head cap allows an easy adaption of the ellipsoid-shaped head cap to the actual humerus shape when implanting the prosthesis. A further benefit is the bone screw component's position being displaced within the humerus head towards the humerus which results in an improved anchoring, due to more bone material and a shorter path through which the articulate forces have to be guided towards the humerus bone. The invention can be used in a method of implanting a humerus head implant. In a first step, the original head of the humerus is resected. In a second step, a stud hole is made into the resected humerus head surface, preferably by a drill or by a reamer. In a third step, a head cap holder, which may be a fixing component comprising a bone screw or any other holding device, is implanted into the humerus head bone. Preferably, the fixing component comprises a conical section fitting to a conical recess in a humerus head cap. A fourth step comprises attachment of a humerus head cap having a half-ellipsoid shape as mentioned above, and a conical recess having a center axis offset to the z-axis of the ellipsoid. A fifth step comprises rotating of the half-ellipsoid head cap into a position which fits to the bone of the humerus head and/or to the glenoid. This last step is optional, as the head cap may have been attached in the correct position and/or direction during the previous step.

### Description of Drawings

In the following the invention will be described by way of example, without limitation of the general inventive concept, on examples of embodiment with reference to the drawings.
Figure 1 shows a humerus head with an implant.
Figure 2 shows a front view of the implant on a humerus.
Figure 3 shows a sectional view along the y-axis.
Figure 4 shows a sectional view along the x-axis.
Figure 5 shows the components of the implant.
Figure 6 shows a fully assembled implant.

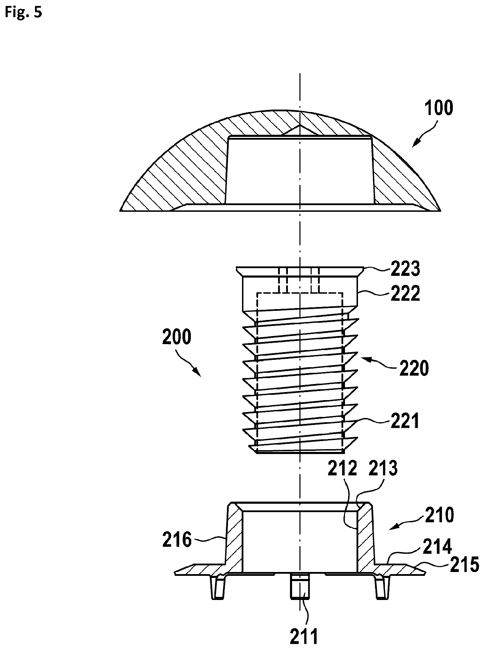

Figure 5 shows the components of the implant in a perspective view.

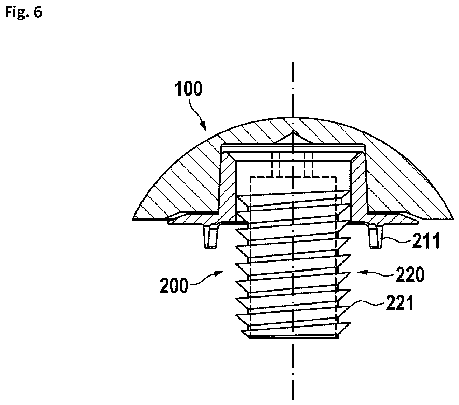

Figure 6 shows a fully assembled implant in a perspective view.

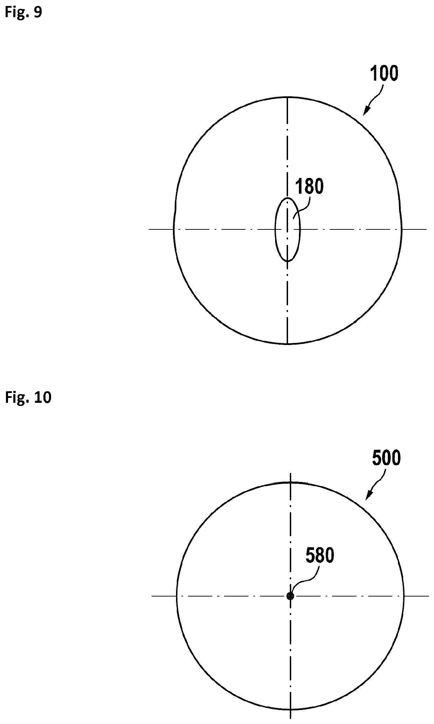

Figure 9 shows the contact surface of the ellipsoid head contacting a glenoid of the shoulder.
Figure 10 shows the contact surface area of a spherical implant head.

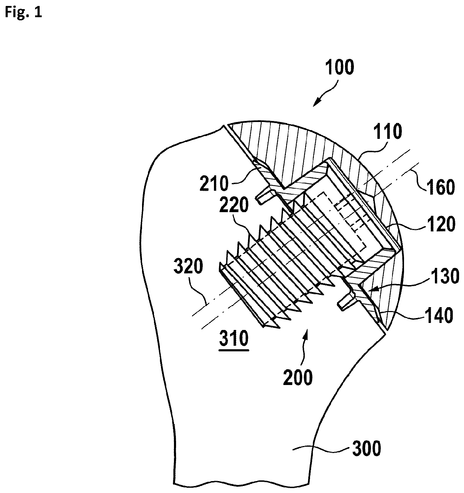

In Figure 1, a humerus head with a humerus head implant is shown. The humerus 300 has a humerus head 310 bearing a head cap 100 by means of a fixing component 200. The head cap 100 comprises an articulating surface 110 which is designed to fit to a glenoid. Opposing to the articulating surface 110 is a conical recess 120 adapted to hold the fixing component 200. There may also be an adapter contact surface 130 opposite to the articulating surface for load bearing, therefore transferring mechanical load from the head cap 100 into the fixing component 200. There may further be a rim 140 at the outside of the adapter contact surface 130. The fixing component may have a threaded bone screw component 220 for anchoring the implant into the bone material of the humerus head 310.

The Figure further shows the center axis 160 of the conical recess which preferably is also the center axis of the fixing component 200 and preferably the center axis of a bone screw component 220. This center axis of the conical recess preferably is offset to the humerus center 320, in a direction towards the shaft of the humerus for an improved load transfer and improved embedding into the humerus bone material. Preferably, the center is related to the resected bone area for placing the implant.

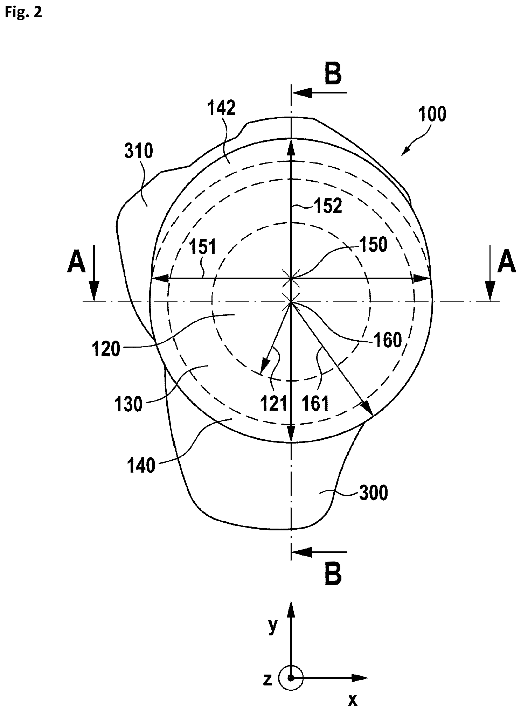

In Figure 2, a front view of the implant on a humerus is shown. A head cap 100 is located on the humerus head 310. For a better orientation, a coordinate system is shown. It has a z-axis extending out of the drawing plane, an x-axis to the right, and a y-axis to the top. The center of the coordinate system is at the center 150 of the ellipsoid-shaped head. The ellipsoid-shaped head has an extension 151 in x-direction (cap width) and an extension 152 in y-direction (cap height), whereas the head height is larger than the head width. The conical recess 120 which is shown here as a dashed line, as it is hidden within the cap, has a radius 121, whereas the outer radius of the rim of adapter contact surface 140 has a radius 161. The center 160 of the conical recess is offset to the bottom or in a negative y-direction from the center 150 of the ellipsoid head. Actually, the ellipsoid head is only a half-ellipsoid, which will be shown later in detail.

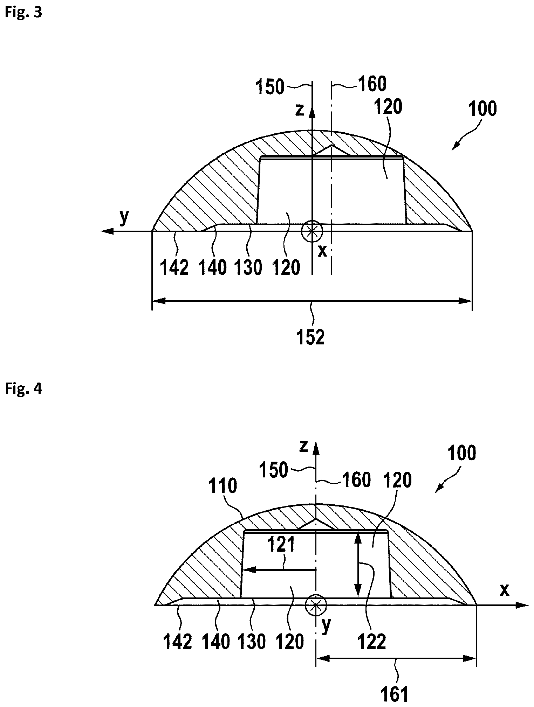

In Figure 3, a sectional view along the y-axis (section B-B of figure 2) is shown. Here, the larger diameter 152 (cap height) is shown from left to right. There is an extension surface 142 at the bottom of the head cap, corresponding to the additional area caused by the larger cap height compared to the cap width. Furthermore, the displacement of the center 160 of the conical recess against the ellipsoid head z-axis 150 is shown. This Figure clearly shows that the ellipsoid-shaped head only extends into a positive z-direction and has no negative z-dimension. Therefore, it is only a half-ellipsoid-shape.

In Figure 4, a sectional view along the x-axis (section A-A of figure 2) is shown. In this view, the center 160 of the conical recess 120 and the ellipsoid head z-axis are behind each other and overlap in the Figure. This Figure shows further dimensions of the conical recess 120. It has a recess height 122 and a recess radius 121. As the recess has a conical shape, the radius 121 is decreasing with increasing z. Accordingly, the radius 121 is largest at the bottom (z = 0), and is decreasing with the height of the ellipsoid shape.

In Figure 5, the components of the implant are shown, comprising a half-ellipsoid head cap 100 and a fixing component 200, which further comprises a head adapter 210 and a bone screw component 220. This not forming part of the invention, the bone screw component 220 may be any means for screwing or holding into the humerus bone material. The head adapter 210 and the bone screw component 220 may also be of one part or they may comprise further components. In this preferred embodiment, the bone screw component has at least one thread 221 to be screwed directly into the humerus bone. It preferably comprises a centering shaft 222 and a limit stop 223. The head adapter 210 preferably has at least one protrusion 211, which may be pressed into the bone material of the humerus and secures the implant against rotating. The head adapter 210 furthermore preferably has a center bore 212 which further preferably has a chamfered bottom section 213. It is preferred, if the center bore 212 is adapted to the centering shaft 222 of the bone screw component, and the chamfer 213 is adapted to fit to the limit stop 223 of the bone screw component. This allows a precise positioning and fixing of the bone screw component to the head adapter. It is further preferred, if the head adapter has a head contact surface 214 which may have a rim 215. This head contact surface may be used for optimized load transfer from the head cap 100 via the head adapter into the bone. Furthermore, it is preferred if the head adapter 210 provides a conical section 216 which preferably fits into the conical recess 120 of the ellipsoid head.

In Figure 6, a fully assembled implant comprising the components as shown in the previous Figure is shown.

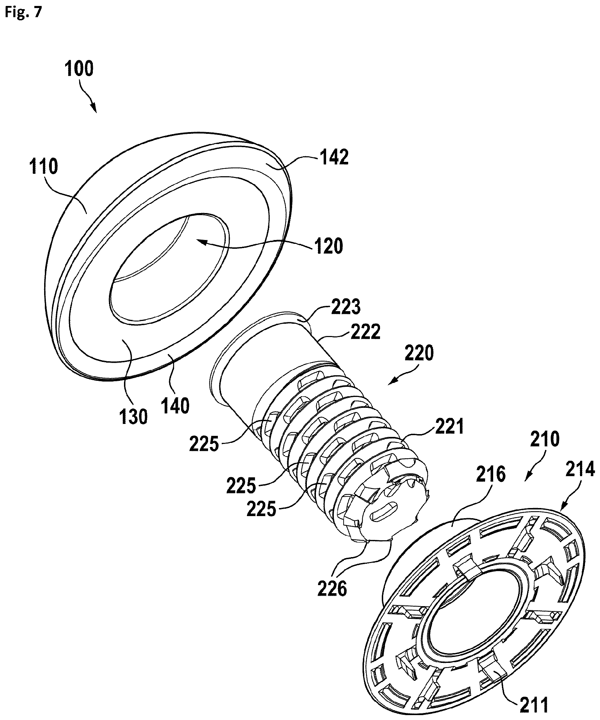

In Figure 7, the components of an implant are shown in a perspective view. This figure shows a modified embodiment with the bone screw component 220 having a plurality of holes 225 which allow ingrowth of bone and improve anchoring. Furthermore, there are cutting edges 226 at the end of the thread 221 which simplify screwing of the screw component into bone material.

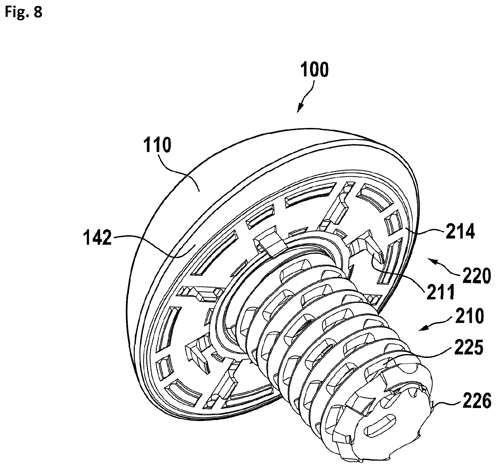

In Figure 8, a fully assembled implant is shown in a perspective view.

In Figure 9, the contact area 180 of the half-ellipsoid head cap 100 is shown when contacting a glenoid of a shoulder. Due to the ellipsoid shape, the glenoid contact surface 180 is comparatively large and has an elliptic shape.

In Figure 10, the contact area 520 of a spherical implant 500 head is shown. Here, the contact area 520 has a circular shape and is significantly smaller than the contact area of an half-ellipsoid head as shown before.

### List of reference numerals

- 100: head cap
- 110: articulating surface
- 120: conical recess
- 121: recess radius
- 122: recess height
- 130: adapter contact surface
- 140: rim of adapter contact surface
- 142: extension surface
- 150: ellipsoid head z-axis
- 151: cap width
- 152: cap height
- 160: center of conical recess
- 161: outer radius of contact surface
- 180: glenoid contact area
- 200: fixing component
- 210: head adapter
- 211: protrusion
- 212: bore
- 213: chamfer
- 214: head contact surface
- 215: rim of head contact surface
- 216: conical section
- 220: bone-screw component
- 221: thread
- 222: centering shaft
- 223: limit stop
- 225: holes
- 226: cutting edges
- 300: humerus
- 310: humerus head
- 320: humerus center
- 500: spherical head
- 520: glenoid contact area of spherical head

## Claims

1. Humerus head implant comprising a head cap (100) and a fixing component (200),
the head cap (100) having a half-ellipsoid shape extending into positive direction of a z-axis, in positive and negative directions of an x-axis, and in positive and negative directions of a y-axis, and the extension of the head cap is largest along the y-axis,
whereas the extension (151) in the direction of the x-axis is in a range of 0.8 to 0.95 of the extension (152) in y-direction, and the head cap (100) further comprises a conical recess having a center axis (160) which is displaced in a negative y-direction with respect to the z-axis, the fixing component comprises a conical section (216) fitting into the conical recess (120) and allowing rotation of the head (100) relative to the fixing component,
the fixing component further comprises a bone-screw component (220).

2. Humerus head implant according to claim 1,
**characterized in that**
the fixing component further comprises a head adapter (210) which interfaces between the screw component (220) and the head (100).

3. Humerus head implant according to claim 2,
**characterized in that**,
the head adapter comprises at least one protrusion (211).

4. Humerus head implant according to any one of the previous claims, wherein the ratio of the extension in x-direction to the extension in y-direction is between 0.85 and 0.95.

5. Humerus head implant according to claim 4,
wherein the ratio of the extension in x-direction to the extension in y-direction is between 0.90 and 0.91.

6. Humerus head implant according to any one of the previous claims, wherein the displacement of the center axis (160) in a negative y-direction with respect to the z-axis is between 3 and 10 mm.

7. Humerus head implant according to claim 6,
wherein the displacement in a negative y-direction with respect to the z-axis is between 4 and 6 mm.

## Patentansprüche

1. Humeruskopfimplantat umfassend eine Kopfkappe (100) und eine Fixierungskomponente (200),
wobei die Kopfkappe (100) eine Halbellipsoid-Form aufweist, die sich in positive Richtung einer z-Achse, in positiver und negativer Richtung einer x-Achse, und in positiver und negativer Richtung einer y-Achse erstreckt, und die Ausdehnung der Kopfkappe entlang der y-Achse am größten ist,
wobei die Ausdehnung (151) in der Richtung der x-Achse in einem Bereich von 0,8 bis 0,95 der Ausdehnung (152) in y-Richtung liegt, und die Kopfkappe (100) weiterhin eine konische Aussparung mit einer Mittelachse (160) umfasst, welche in negativer y-Richtung in Bezug auf die z-Achse verschoben ist, die Fixierungskomponente einen konischen Abschnitt (216) umfasst, der in die konische Vertiefung (120) passt und eine Drehung des Kopfes (100) relativ zu der Fixierungskomponente ermöglicht,
wobei die Fixierungskomponente weiterhin eine Knochenschraubenkomponente (220) umfasst.

2. Humeruskopfimplantat nach Anspruch 1,
**dadurch gekennzeichnet, dass**
die Fixierungskomponente ferner einen Kopfadapter (210) umfasst, der zwischen der Schraubenkomponente (220) und dem Kopf (100) verbindet.

3. Humeruskopfimplantat nach Anspruch 2,
**dadurch gekennzeichnet, dass**
der Kopfadapter mindestens einen Vorsprung (211) umfasst.

4. Humeruskopfimplantat nach einem der vorhergehenden Ansprüche,
wobei das Verhältnis der Ausdehnung in x-Richtung zur Ausdehnung in y-Richtung zwischen 0,85 und 0,95 beträgt.

5. Humeruskopfimplantat nach Anspruch 4,
wobei das Verhältnis der Ausdehnung in x-Richtung zur Ausdehnung in y-Richtung zwischen 0,90 und 0,91 beträgt.

6. Humeruskopfimplantat nach einem der vorhergehenden Ansprüche,
wobei die Verschiebung der Mittelachse (160) in einer negativen y-Richtung in Bezug auf die z-Achse zwischen 3 und 10 mm beträgt.

7. Humeruskopfimplantat nach Anspruch 6,
wobei die Verschiebung in einer negativen y-Richtung in Bezug auf die z-Achse zwischen 4 und 6 mm beträgt.

## Revendications

1. Implant de tête humérale comprenant une calotte de tête (100) et un composant de fixation (200),
la calotte de tête (100) ayant une forme semi-ellipsoïdale qui s'étend dans la direction positive d'un axe z, dans les directions positive et négative d'un axe x et dans les directions positive et négative d'un axe y et l'étendue de la calotte de tête étant maximale le long de l'axe y,
l'étendue (151) dans la direction de l'axe x étant comprise entre 0,8 et 0,95 fois l'étendue (152) dans la direction de l'axe y, et
la calotte de tête (100) comprenant en outre un renfoncement conique avec un axe central (160) qui est décalé dans la direction négative de l'axe y par rapport à l'axe z,
le composant de fixation comprenant une partie conique (216) qui s'adapte dans le renfoncement conique (120) et permet la rotation de la tête (100) par rapport au composant de fixation,
le composant de fixation comprenant en outre un composant formant vis à os (220).

2. Implant de tête humérale selon la revendication 1, **caractérisé en ce que** le composant de fixation comprend en outre un adaptateur de tête (210) qui s'interface entre le composant formant vis à os (220) et la tête (100).

3. Implant de tête humérale selon la revendication 2, **caractérisé en ce que** l'adaptateur de tête comporte au moins une saillie (211).

4. Implant de tête humérale selon l'une quelconque des revendications précédentes, dans lequel le rapport entre l'étendue sur l'axe x et l'étendue sur l'axe y est compris entre 0,85 et 0,95.

5. Implant de tête humérale selon la revendication 4, dans lequel le rapport entre l'étendue sur l'axe x et l'étendue sur l'axe y est compris entre 0,90 et 0,91.

6. Implant de tête humérale selon l'une quelconque des revendications précédentes, dans lequel le décalage de l'axe central (160) dans une direction négative sur l'axe y par rapport à l'axe z est compris entre 3 et 10 mm.

7. Implant de tête humérale selon la revendication 6, dans lequel le décalage dans une direction négative sur l'axe y par rapport à l'axe z est compris entre 4 et 6 mm
